# EUROPÄISCHE PATENTANMELDUNG

(11) **EP 2 206 447 A1**
(43) Veröffentlichungstag der Anmeldung: **14.07.2010**
(21) Anmeldenummer: 09015282.8
(22) Anmeldetag: 10.12.2009
(51) Int. Cl.: A45D 34/04

(54) **Vorrichtung zum Auftragen einer Creme**

(30) Priorität: 13.01.2009 DE 202009000352 U; 28.01.2009 DE 202009001053 U
(71) Anmelder: Neukamp, Benno, 83471 Berchtesgaden (DE)
(72) Erfinder: Neukamp, Benno, 83471 Berchtesgaden (DE)
(74) Vertreter: Haft, von Puttkamer, Berngruber, Karakatsanis

(57) **Zusammenfassung**

Die Erfindung betrifft eine Vorrichtung zum Auftragen eines Einreibemittels auf dem Rücken oder anderen Körperteilen einer Person durch einen Schwamm (1), der an einem Schwammträger (3) vorgesehen ist, der eine Verlängerung (4) aufweist, die an ihrem Ende mit einem Griff (5, 20, 21) versehen ist.

## Beschreibung

Die Erfindung bezieht sich auf eine Vorrichtung zum Auftragen einer Creme oder dergleichen Einreibemittel auf dem Rücken oder anderen Körperteilen einer Person.

Da der gesamte Rückenbereich einer Person von ihr mit der Hand schwer erreichbar ist, hat sie Mühe ihren Rücken vollständig einzucremen.

Aufgabe der Erfindung ist es daher, das Auftragen einer Creme oder eines anderen Mittels auf den Rücken aber auch an anderen nicht gut erreichbaren Körperteilen zu erleichtern.

Dies wird erfindungsgemäß mit einer Vorrichtung erreicht, die einen Schwamm aufweist, auf die die Creme oder sonstige Einreibemittel aufgetragen wird, wobei der Schwamm an einem Schwammträger vorgesehen ist, der eine Verlängerung aufweist, die an ihrem anderen Ende mit einem Griff versehen ist.

Mit der erfindungsgemäßen Vorrichtung kann eine Creme oder dergleichen fließfähiges Material aufgetragen werden, das von dem Schwamm aufgenommen wird. Die Creme kann z.B. eine Sonnencreme oder eine andere Körperpflegecreme oder eine medizinische Creme sein. Das fließfähige Mittel kann jedoch auch beispielsweise eine Bodylotion, ein Sonnenöl oder dergleichen Flüssigkeit sein. Auch kann das Einreibemittel gegebenenfalls pulverförmig sein.

Der Schwamm kann an dem Schwammträger lösbar befestigbar sein. Damit kann für die jeweiligen Einreibemittel der optimale Schwamm verwendet werden. So kann z.B. für eine härtere Creme ein härterer Schwamm verwendet werden. Die unterschiedlichen Schwämme können beispielsweise durch unterschiedliche Farben gekennzeichnet werden.

Die Verlängerung kann mit dem Schwammträger fest verbunden sein. Vorzugsweise ist sie jedoch am Schwammträger befestigbar, also mit dem Schwammträger lösbar verbunden.

Die Verlängerung kann in unterschiedlicher Weise ausgebildet sein. So kann sie beispielsweise durch zwei Bänder, Schnüre oder dergleichen Zugmittel gebildet sein, die mit dem Schwammträger verbunden sind und an ihren Ende jeweils einen Abschnitt aufweisen, der als Griff dient. Die Person, die sich den Rücken eincremen möchte, ergreift nach dem Auftragen des Einreibemittels auf den Schwamm die beiden Zugmittel am Griff und zieht dann die beiden Zugmittel unter Spannung hin und her, um das Einreibemittel auf dem Schwamm auf ihren Rücken zu reiben.

Darüberhinaus wird als Verlängerung insbesondere ein Stiel verwendet. Der Stiel kann dabei verlängerbar ausgebildet sein, beispielsweise durch Abknicken oder eine teleskopische Ausbildung. Wenn der Stiel zudem mit dem Schwammträger lösbar verbunden ist, kann auf diese Weise die Vorrichtung so weit zusammengelegt werden, dass sie in einer kleinen Tasche Platz findet.

Aus ergonomischen Gründen hat es sich zudem als vorteilhaft erwiesen, den Stil gebogen oder in anderer Weise gekrümmt auszubilden, um das Einreibemittel auf dem Schwamm großflächig auftragen zu können, ohne sich verrenken zu müssen.

In weiterer Ausgestaltung der Erfindung kann der Schwamm hin- und her schwingend ausgebildet sein, beispielsweise mit einem Elektromotor mit Getriebe, der an dem Stiel oder dem Schwammträger vorgesehen ist. Zum Antrieb des Elektromotors kann z.B. der Stiel einen Akku oder einen Netzanschluss aufweisen.

Vorzugsweise weist der Schwamm an seiner dem Schwammträger zugewandten Seite eine Abdichtung auf, beispielsweise eine Gummi- oder Kunststoffschicht, um zu verhindern, dass das Einreibemittel auf den Schwammträger gelangt.

Der Schwammträger kann in unterschiedlicher Weise ausgebildet sein. So kann er beispielsweise aus einem zylindrischen oder prismenförmigen Kern bestehen, um den der z.B. schlauchförmig ausgebildete Schwamm gelegt wird.

Stattdessen kann der Schwammträger auch plattenförmig ausgebildet sein, wobei der Schwamm dann auf einer Seite des plattenförmigen Schwammträgers befestigt oder lösbar befestigbar sein kann.

Die Abdichtung kann dann durch eine Platte gebildet werden. Zur lösbaren Befestigung des Schwamms an dem Schwammträger kann bei dieser Ausführungsform der Schwammträger eine Ausnehmung aufweisen, in die die Platte mit dem Schwamm schiebbar ist, beispielsweise durch zwei gegenüberliegende Führungsnuten in der Ausnehmung, in die die Platte an dem Schwamm mit ihrer Längskante eingreift.

Nachstehend ist die erfindungsgemäße Vorrichtung anhand der beigefügten Zeichnung beispielhaft näher erläutert. Darin zeigen jeweils schematisch:
Figur 1 und 2 eine perspektivische Ansicht bzw. eine Seitenansicht einer Ausführungsform der Vorrichtung mit zum Teil weggebrochenem Stiel;
Figur 3 eine perspektivische Ansicht einer anderen Ausführungsform des Stiels der Vorrichtung; und
Figur 4 eine Draufsicht auf eine andere Ausführungsform der Vorrichtung.

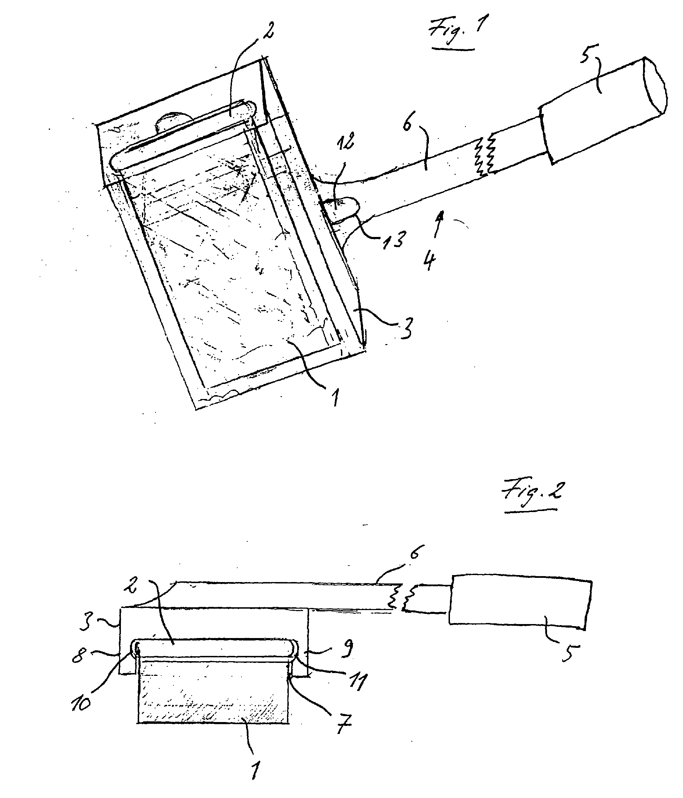

Gemäß Figur 1 und 2 weist die Vorrichtung einen rechteckigen Schwamm 1 zur Aufnahme einer Creme oder dergleichen auf, der an einer Platte 2 z.B. aus Kunststoff befestigt ist. Der Schwamm 1 mit der Platte 2 ist an einem plattenförmigen Träger 3 befestigt.

Die Vorrichtung ist mit einem Handgriff 5 betätigbar. Als Verlängerung 4 des Handgriffs 5 ist ein Stiel 6 vorgesehen, der an dem Schwammträger 3 lösbar befestigbar ist.

Zur lösbaren Befestigung des Schwamms 1 der Platte 2 an dem Schwammträger 3 ist der Schwammträger 3 an der dem Schwamm 1 zugewandten Seite mit einer Längsausnehmung 7 versehen. An den gegenüberliegenden Seitenwänden 8, 9 der Ausnehmung 7 sind innen Führungsnuten 10, 11 vorgesehen, in die Platte 2 mit ihren Längskanten schiebbar ist. Zur Befestigung des Stiels 6 an dem Schwammträger 3 kann der Schwammträger 3 einen Vorsprung 12 aufweisen, der in eine Ausnehmung 13 in dem Stiel 6 steckbar ist.

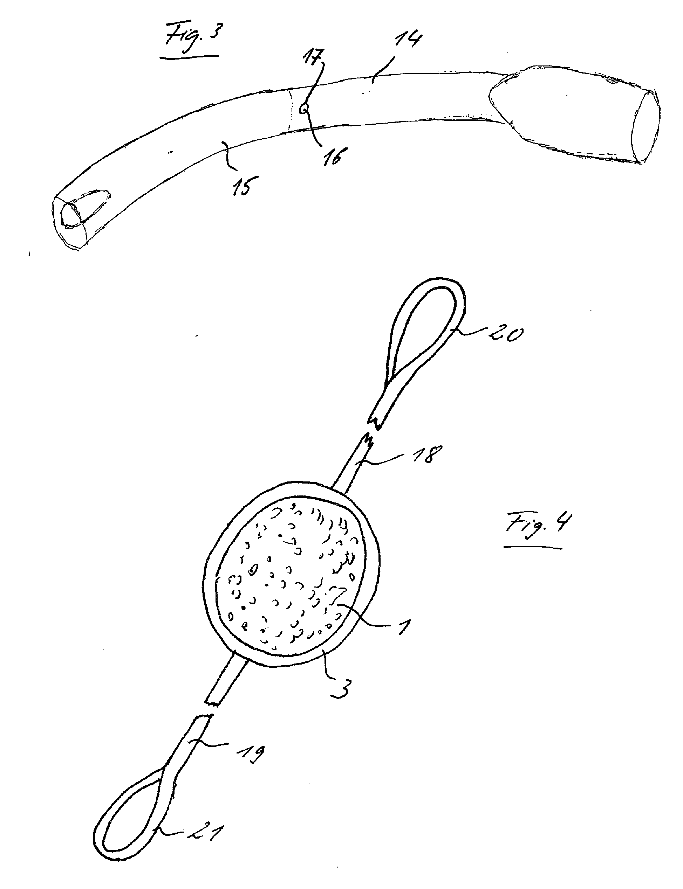

Gemäß Figur 3 ist der Stiel 6 gebogen und zudem teleskopierbar ausgebildet. D.h. er weist ein gekrümmtes Außenohr 14 auf, in das das Innenteil 15 gleicher Krümmung schiebbar ist. Zur Arretierung des Innenteils 15 in dem Außenrohr 14 ist an dem Innenteil 15 ein federbelasteter Knopf 16 vorgesehen, der in eine Rastausnehmung 17 in dem Außenrohr 14 einrastbar ist.

Bei der Ausführungsform nach Figur 4 ist der Schwamm 1 in einem schalenförmigen Schwammträger 3 befestigt. An dem Schwammträger 3 sind zwei z.B. als Bänder ausgebildete Zugmittel 18 und 19 befestigt, die an ihrem freien Ende jeweils mit einem Handgriff 20, 21 versehen sind, der als Schlaufe ausgebildet ist.

## Patentansprüche

1. Vorrichtung zum Auftragen eines Einreibemittel auf dem Rücken oder anderen Körperteilen einer Person, **gekennzeichnet durch** einen Schwamm (1), der an einem Schwammträger (3) vorgesehen ist, der eine Verlängerung (4) aufweist, die an ihrem Ende mit einem Griff (5, 20, 21) versehen ist.

2. Vorrichtung nach Anspruch 1, **dadurch gekennzeichnet, dass** die Verlängerung (4) an dem Schwammträger (3) befestigt oder an dem Schwammträger (3) lösbar befestigbar ausgebildet ist.

3. Vorrichtung nach Anspruch 1 oder 2, **dadurch gekennzeichnet, dass** die Verlängerung (4) durch einen Stiel (6) gebildet wird.

4. Vorrichtung nach Anspruch 1 oder 2, **dadurch gekennzeichnet, dass** die Verlängerung (4) durch zwei mit dem Schwammträger (3) verbundene Zugmittel (18, 19) mit jeweils einen Griff (20, 21) am von dem Schwammträger (3) abgewandten Ende der Zugmittel (20, 21) gebildet wird.

5. Vorrichtung nach Anspruch 3, **dadurch gekennzeichnet, dass** der Stiel (6) verlängerbar ausgebildet ist.

6. Vorrichtung nach Anspruch 3 oder 5, **dadurch gekennzeichnet, dass** der Stiel (6) gekrümmt ausgebildet ist.

7. Vorrichtung nach Anspruch 1, **dadurch gekennzeichnet, dass** der Schwamm (1) am Schwammträger (3) lösbar befestigbar ist.

8. Vorrichtung nach Anspruch 1, **dadurch gekennzeichnet, dass** der Schwamm (1) an der dem Schwammträger (3) zugewandten Seite mit einer Abdichtung versehen ist.

9. Vorrichtung nach Anspruch 8, **dadurch gekennzeichnet, dass** die Abdichtung durch eine Platte (2) gebildet wird.

10. Vorrichtung nach Anspruch 7 und 9, **dadurch gekennzeichnet, dass** der Schwammträger (3) zur lösbaren Befestigung des Schwamms (1) eine Ausnehmung (7) aufweist, in die die Platte (2) mit dem Schwamm (1) schiebbar ist.

11. Vorrichtung nach Anspruch 3, **dadurch gekennzeichnet, dass** der Stiel (6) oder der Schwammträger (3) zum Schwingen des Schwamms (1) mit einem Elektromotor mit Getriebe versehen ist.
